**Europäisches Patentamt**

**European Patent Office**

**Office européen des brevets**

(19)

(11) Numéro de publication: **0 170 569**
**A1**

(12) # DEMANDE DE BREVET EUROPEEN

(21) Numéro de dépôt: **85401332.3**

(22) Date de dépôt: **01.07.85**

(51) Int. Cl.⁴: **A 61 M 5/14**

(30) Priorité: **04.07.84 FR 8410602**

(43) Date de publication de la demande:
**05.02.86 Bulletin 86/6**

(84) Etats contractants désignés:
**AT BE CH DE FR GB IT LI LU NL SE**

(71) Demandeur: **M M S S.A.**
**89-93, avenue Paul-Vaillant Couturier**
**F-94250 Gentilly(FR)**

(72) Inventeur: **Siretchi, Roman**
**94, rue Haxo**
**F-75020 Paris(FR)**

(74) Mandataire: **Chameroy, Claude et al,**
**c/o Cabinet Malemont 42, avenue du Président Wilson**
**F-75116 Paris(FR)**

(54) **Dispositif de pompage à contrôle de débit, notamment pour la nutrition entérale ou parentérale.**

(57) Dispositif de pompage à contrôle de débit, notamment pour la nutrition entérale ou parentérale, comprenant un système de propulsion de fluide, constitué d'une tubulure (T) et d'un moyen de pompage (SP), et un système de mesure de débit (SM) inséré dans la tubulure en aval du moyen de pompage (SP) et en amont du circuit d'utilisation du fluide, caractérisé en ce que le système de mesure de débit (SM) est constitué de deux circuits en parallèle ($B_1$, $B_2$, $B_3$, $B_4$) reliés par un conduit de dérivation (C) dans lequel est disposé un piston (P) apte à se déplacer librement, ledit système de mesure (SM) comprenant en outre:

- quatre vannes ($EV_1$, $EV_2$) disposées sur les deux circuits en parallèle, respectivement en amont et en aval du conduit de dérivation (C);

- des moyens (Q) pour commander le fonctionnement en synchronisme et deux par deux des quatre vannes ($EV_1$, $EV_2$), de manière à faire circuler le fluide alternativement dans un sens et dans l'autre à l'intérieur du conduit de dérivation (C);

- des moyens (DA, DB) pour détecter l'arrivée du piston (P) entraîné par le fluide aux deux extrémités (A, B) du conduit de dérivation (C) et commander en conséquence l'inversion du sens de circulation du fluide dans ce conduit par l'intermédiaire de quatre vannes;

- des moyens (CP) pour mesurer l'intervalle de temps entre deux détections; et

- des moyens (PL) pour commander le système de propulsion de fluide (SP) en fonction de l'intervalle de temps ainsi mesuré et de la valeur du débit programmé souhaité.

./...

EP 0 170 569 A1

1

## Dispositif de pompage à contrôle de débit, notamment pour la nutrition entérale ou parentérale

La présente invention concerne un dispositif de pompage à contrôle de débit, notamment pour la nutrition entérale ou parentérale, comprenant un système de propulsion de fluide, constitué d'une tubulure et d'un moyen de pompage, et un système de mesure de débit inséré dans la tubulure en aval du moyen de pompage et en amont du circuit d'utilisation du fluide.

On sait que les pompes employées dans le domaine médical pour la nutrition entérale ou parentérale doivent avoir un débit aussi constant et précis que possible. A cet effet, on utilise plusieurs types de pompes.

Dans les pompes dites à compte-gouttes, le contrôle du débit s'effectue par le comptage des gouttes dans l'unité de temps. De telles pompes ont une précision toute relative, car le volume des gouttes varie en fonction de la nature du fluide à perfuser. De plus, ces pompes ne sont utilisables que jusqu'a un certain débit, car au-delà, la détection des gouttes devient impossible.

On connaît également les pompes péristaltiques, à compression ou à traction, fonctionnant par déformation d'un tube calibré dans lequel passe le fluide à perfuser. La précision de ces pompes est relativement bonne, mais dépend essentiellement des caractéristiques du tube déformable. De plus, il n'y a pas de contrôle direct sur le débit obtenu, la pompe étant réglée au départ pour obtenir en sortie un débit présumé fonction du tube calibré utilisé.

Enfin, on utilise aussi des pompes à cassette dont le principe est analogue à celui d'un pousse-seringue. La précision de ces pompes est très bonne, mais elles sont relativement chères. De plus, le débit n'est pas continu et présente des variations cycliques en fonction des mouvements de la cassette.

La présente invention a pour but principal de remédier à ces inconvénients et, pour ce faire, elle a pour objet un dispositif de pompage du type susmentionné qui se caractérise essentiellement en ce que le système de mesure de débit est constitué de deux circuits en parallèle reliés par un conduit de dérivation dans lequel est disposé un piston apte à se déplacer librement, ledit système de mesure comprenant en outre :

- quatre vannes disposées sur les deux circuits en parallèle, respectivement en amont et en aval du conduit de dérivation ;

- des moyens pour commander le fonctionnement en synchronisme de deux par deux des quatre vannes, de manière à faire circuler le fluide alternativement dans le sens et dans l'autre à l'intérieur du conduit de dérivation ;

- des moyens pour détecter l'arrivée du piston entraîné par le fluide aux deux extrémités du conduit de dérivation et commander en conséquence l'inversion du sens de circulation du fluide dans ce conduit par l'intermédiaire des quatre vannes ;

- des moyens pour mesurer l'intervalle de temps entre deux détections ; et

- des moyens pour commander le système de propulsion de fluide en fonction de l'intervalle de temps ainsi mesuré et de la valeur du débit programmé souhaité.

On dispose ainsi d'un moyen extrêmement simple et efficace pour contrôler le débit effectivement délivré par le système de propulsion de fluide et agir en conséquence si besoin est. Avec un tel dispositif de pompage, le débit est donc parfaitement constant et indépendant du système de propulsion de fluide.

De préférence, le conduit de dérivation est constitué par un tube rigide calibré.

C'est en effet le volume du conduit de dérivation qui va déterminer le débit. Toutefois, on pourrait également utiliser un tube non calibré et procéder par étalonnage.

Dans une forme de réalisation particulière de l'invention, les deux circuits en parallèle sont constitués par des tubes souples, sur lesquels agissent quatre vannes du type électrovanne à pincement.

On peut ainsi facilement remplacer après usage toutes les canalisations du système de mesure de débit, ce qui est nécessaire dans les applications médicales pour des raisons de stérilité.

Quant au système de propulsion de fluide, il est avantageusement constitué par une pompe péristaltique, pour les mêmes raisons.

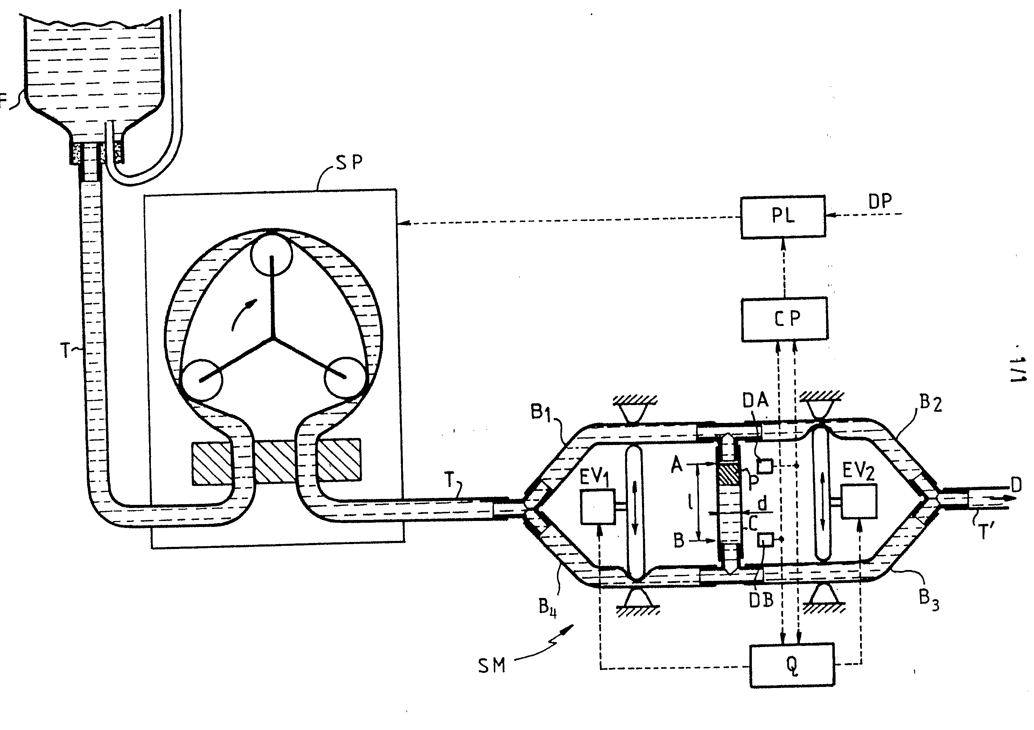

Une forme d'exécution de l'invention est décrite ci-après à titre d'exemple, en référence au dessin annexé dans lequel la figure unique est une vue schématique d'un dispositif de pompage conforme à la présente invention.

Ce dispositif de pompage se compose essentiellement d'un système de propulsion de fluide SP et d'un système de mesure de débit SM. Le système de propulsion de fluide SP est constitué ici d'une pompe péristaltique du type à traction, dont la tubulure déformable T est reliée à une extrémité à un réservoir de fluide en forme de flacon F et à l'autre extrémité à l'entrée du système

3

de mesure de débit SM. La sortie de ce système de mesure de débit est reliée par une tubulure T' au circuit d'utilisation constitué par exemple par un organe de perfusion.

Le système de mesure de débit SM, inséré entre les tubulures T et T', est essentiellement constitué de deux circuits en parallèle reliés l'un à l'autre par un conduit de dérivation C, formant ainsi quatre bras distincts $B_1$, $B_2$, $B_3$ et $B_4$. Ces quatre bras sont constitués par des tronçons de tube souple connectés aux tubulures T et T' ou au conduit de dérivation C par des raccords en T ou en Y. Quant au conduit de dérivation C, il est constitué par un tube rigide calibré à l'intérieur duquel est disposé un piston P apte à se déplacer librement sous l'action du fluide, entre deux points A et B.

Dans les quatre bras $B_1$ à $B_4$ sont disposées des électrovannes du type à pincement agissant par serrage du tube souple constituant lesdits bras. En fait, dans l'exemple particulier décrit ici, on utilise de façon avantageuse deux électrovannes à double voie $EV_1$ et $EV_2$. L'électrovanne $EV_1$ agit sur les bras $B_1$ et $B_4$, et elle est conçue de manière à ouvrir le bras $B_1$ lorsque le bras $B_4$ est fermé, et réciproquement. L'électrovanne $EV_2$ agit de la même manière sur les bras $B_2$ et $B_3$, mais elle est commandée en opposition par rapport à l'électrovanne $EV_1$.

Par ailleurs, deux détecteurs DA et DB, par exemple de type optique, sont respectivement placés au niveau des points A et B du tube calibré C, afin de détecter l'arrivée du piston P en ces points. Les signaux électriques issus de ces deux détecteurs sont tout d'abord appliqués à une bascule bistable Q assurant le fonctionnement en synchronisme des deux électrovannes, et également à un compteur CP dont le signal de sortie est appliqué à un processeur logique PL capable de commander le fonctionnement de la pompe péristaltique.

Le dispositif de pompage qui vient d'être décrit fonctionne de la manière suivante :

Supposons qu'au départ, les électrovannes $EV_1$ et $EV_2$ soient dans la position représentée sur la figure, c'est-à-dire que les bras $B_1$ et $B_3$ soient ouverts et que les bras $B_2$ et $B_4$ soient fermés. Le fluide poussé par la pompe dans la tubulure T va alors obligatoirement passer par le bras $B_1$, puis dans le tube calibré C et enfin par le bras $B_3$ pour parvenir dans la tubulure T'. Le piston P placé à l'intérieur du tube calibré va ainsi se trouver entraîné par le fluide du point A vers le point B.

4

Lorsque le piston arrive au point B, le détecteur DB délivre un signal électrique qui fait changer d'état la bascule Q. La position des électrovannes $EV_1$ et $EV_2$ est alors inversée. Autrement dit, ce sont maintenant les bras $B_1$ et $B_3$ qui sont fermés et les bras $B_2$ et $B_4$ qui sont ouverts. Sous l'action du fluide délivré par la pompe, le piston P va donc se déplacer cette fois-ci de B vers A.

Quand le piston arrive au point A, le détecteur DA délivre un signal qui ramène les électrovannes $EV_1$ et $EV_2$ dans leur position de départ par l'intermédiaire de la bascule Q, et le processus recommence.

On notera dès maintenant que le flux régrograde, qui peut être très gènant dans certains cas, est complètement éliminé grâce à l'inertie de la pompe et au fait que les ouvertures et fermetures des bras se font en synchronisme.

Le compteur CP mesure l'intervalle de temps entre les impulsions engendrées par les deux détecteurs DA et DB, et délivre ainsi un signal de sortie qui est représentatif du débit D dans la tubulure T'. En utilisant un tube calibré C dont on connaît parfaitement le diamètre d et la longueur 1 séparant les points A et B, on peut donc facilement déterminer la valeur de ce débit D.

Cette opération est réalisée par le processeur logique PL dans lequel on aura préalablement enregistré le débit programmé DP souhaité. Le débit D ainsi calculé est alors comparé au débit programmé DP et s'il est inférieur, le processeur commande l'accélération du moteur d'entraînement de la pompe péristaltique. Dans le cas contraire, il provoque un ralentissement dudit moteur d'entraînement. Le débit délivré est ainsi constant et uniforme.

La précision d'un tel dispositif de pompage est dictée par les caractéristiques du tube calibré C et est indépendante des dimensions des autres tubulures, notamment de la tubulure T associée à la pompe péristaltique, qui n'a donc pas besoin d'être calibrée. Ceci diminue sensiblement le coût d'utilisation du dispositif. On sait en effet que dans les applications médicales il est nécessaire de jeter après usage tous les éléments qui ont été en contact avec le fluide à transfuser.

Pour changer la gamme de débit tout en conservant la même précision, il suffit de modifier le diamètre d et/ou la longueur 1 du tube calibré C. Le dispositif de pompage selon l'invention présente donc également une très grande souplesse d'utilisation.

0170569

5

Il va de soi par ailleurs que les deux électrovannes à double voie
$EV_1$ et $EV_2$ pourraient être remplacées par deux électrovannes à trois voies
insérées au niveau des embranchements avec les tubulures T et T', ou au
niveau des embranchements avec le conduit de dérivation C, à la place des
raccords en T ou en Y correspondants.

Revendications

1. Dispositif de pompage à contrôle de débit, notamment pour la nutrition entérale ou parentérale, comprenant un système de propulsion de fluide, constitué d'une tubulure (T) et d'un moyen de pompage (SP), et un système de mesure de débit (SM) inséré dans la tubulure en aval du moyen de pompage (SP) et en amont du circuit d'utilisation du fluide, caractérisé en ce que le système de mesure de débit (SM) est constitué de deux circuits en parallèle ($B_1$ $B_2$, $B_3$ $B_4$) reliés par un conduit de dérivation (C) dans lequel est disposé un piston (P) apte à se déplacer librement, ledit système de mesure (SM) comprenant en outre :

- quatre vannes ($EV_1$, $EV_2$)disposées sur les deux circuits en parallèle, respectivement en amont et en aval du conduit de dérivation (C) ;

- des moyens (Q) pour commander le fonctionnement en synchronisme et deux par deux des quatre vannes ($EV_1$, $EV_2$), de manière à faire circuler le fluide alternativement dans un sens et dans l'autre à l'intérieur du conduit de dérivation (C) ;

- des moyens (DA, DB) pour détecter l'arrivée du piston (P) entraîné par le fluide aux deux extrémités (A, B) du conduit de dérivation (C) et commander en conséquence l'inversion du sens de circulation du fluide dans ce conduit par l'intermédiaire des quatre vannes ;

- des moyens (CP) pour mesurer l'intervalle de temps entre deux détections; et

- des moyens (PL) pour commander le système de propulsion de fluide (SP) en fonction de l'intervalle de temps ainsi mesuré et de la valeur du débit programmé souhaité.

2. Dispositif de pompage selon la revendication 1, caractérisé en ce que le conduit de dérivation (C) est constitué par un tube rigide calibré.

3. Dispositif de pompage selon la revendication 1 ou 2, caractérisé en ce que les deux circuits en parallèle ($B_1$ $B_2$, $B_3$,$B_4$) sont constitués par des tubes souples, sur lesquels agissent quatre vannes ($EV_1$, $EV_2$) du type électrovanne à pincement.

4. Dispositif selon la revendication 3, caractérisé en ce qu'il ne comprend que deux électrovannes à pincement($EV_1$, $EV_2$) du type à double voie susceptible d'agir alternativement - l'une en amont du conduit de dérivation et l'autre en aval dudit conduit - sur chacun des deux circuits en parallèle.

5. Dispositif selon la revendication 3, caractérisé en ce qu'il ne comprend que deux électrovannes à pincement du type à triple voie susceptible d'agir alternativement - l'une en amont du conduit de dérivation et l'autre en aval dudit conduit - sur chacun des deux circuits en parallèle et insérés au niveau des embranchements des deux circuits en parallèle avec d'une part la tubulure en sortie du moyen de pompage et d'autre part la tubulure du circuit d'utilisation du fluide, ou au niveau des embranchements des deux circuits en parallèle avec le conduit de dérivation.

6. Dispositif de pompage selon l'une quelconque des revendications 1 à 2, caractérisé en ce que le moyen de pompage (SP) du système de propulsion de fluide est constitué par une pompe péristaltique.

Office européen
des brevets

**RAPPORT DE RECHERCHE EUROPEENNE**

0170569

Numero de la demande

EP 85 40 1332

## DOCUMENTS CONSIDERES COMME PERTINENTS

| Catégorie | Citation du document avec indication. en cas de besoin. des parties pertinentes | Revendication concernee | CLASSEMENT DE LA DEMANDE (Int Cl 4) |
|---|---|---|---|
| | | | A 61 M 5/14 |
| A | DE-A-2 330 382 (ALFA, LAVAL) <br> * Pages 5,6 et 7; figure 1 * | 1,2 | |
| | --- | | |
| A | FR-A-1 407 789 (G. LOBBE) <br><br> * Colonne 2, lignes 11-33; figures 1,2 * | 1,2,3, 4,5 | |
| | --- | | |
| A | US-A-3 835 874 (F. DELLASALA) <br> * Colonne 2, lignes 58-67; figure 1 * | 1,2 | |
| | --- | | |
| A | DE-A-2 816 924 (M. HUBERT) <br> * Colonne 2; revendication 8; figure 1 * | 6 | |
| | --- | | DOMAINES TECHNIQUES RECHERCHES (Int. Cl 4) |
| A | FR-A- 745 683 (L. ROUX) <br> * Page 1, lignes 6-25; figure 2 * | 1,2 | A 61 M <br> F 02 B |
| | --- | | F 04 B |
| A | GB-A-1 051 710 (DANFOSS) <br> * Page 2, lignes 10-60; figure 1 * | 1,2,3 | G 01 F |
| | ----- | | |

Le présent rapport de recherche a été établi pour toutes les revendications

| Lieu de la recherche <br> LA HAYE | Date d'achèvement de la recherche <br> 01-10-1985 | Examinateur <br> EHRSAM F.J.A. |
|---|---|---|